# EUROPEAN PATENT APPLICATION

(11) **EP 4 534 960 A1**
(43) Date of publication of application: **09.04.2025**
(21) Application number: 24204481.6
(22) Date of filing: 03.10.2024
(51) Int. Cl.: G01F 23/296, A62C 37/50, G01N 29/02, G01N 29/24, G01N 33/00

(54) **POWDER LEVEL DETECTION SYSTEM**

(30) Priority: 03.10.2023 US 202318376295
(71) Applicant: Kidde Technologies, Inc., Wilson, NC 27896 (US)
(72) Inventor: ANGLE, Jeffrey, Knightdale, 27545 (US); BALDWIN, Eli, Knightdale, 27545 (US)
(74) Representative: Dehns

(57) **Abstract**

An assembly for detecting agglomeration of a powder for a fire suppression system includes a vessel configured to contain a powder and a compressed gas and a plurality of piezoelectric sensors disposed in the vessel. At least one sensor of the plurality of sensors is disposed at an interface of the powder and the compressed gas when the vessel is in a filled state.

## Description

### BACKGROUND

The present disclosure relates generally to fire suppression systems and more particularly to monitoring a dry powder fire suppression agent in a storage vessel.

Dry chemical agents having low ozone depletion potential (ODP) and low global warming potential (GWP) and systems that utilize such dry chemical agents are being developed to replace halon-based agents and other environmentally damaging agents used for fire suppression in aerospace and other applications. Dry powders can agglomerate in the process of pressurizing storage vessels and can also agglomerate in storage vessels over time and/or with changes in pressure or temperature. The agglomerated powder can clog dispensing nozzles and/or upstream conduits. The agglomeration of the dry powder agent can result in insufficient discharge of the dry powder agent into a protected space during a fire event thereby rendering the fire suppression system ineffective.

A need exists for detecting agglomeration of the dry powder agents in storage to ensure that the dry powder agent is available for fire suppression.

### SUMMARY

An assembly for detecting agglomeration of a powder for a fire suppression system includes a vessel configured to contain a powder and a compressed gas and a plurality of piezoelectric sensors disposed in the vessel. At least one sensor of the plurality of sensors is disposed at an interface of the powder and the compressed gas when the vessel is in a filled state.

A dry powder fire suppressant monitoring system includes a vessel configured to contain a powder and a compressed gas, a plurality of piezoelectric sensors disposed in the vessel, and a controller communicatively coupled to the plurality of sensors. At least one sensor of the plurality of sensors is disposed at an interface of the powder and the compressed gas when the vessel is in a filled state. The controller is configured to receive an output from the plurality of sensors that corresponds to powder load, indicating at least one of a presence, a volume level, and a condition of the powder in the vessel, and the controller configured to indicate when a powder volume level falls below a minimum threshold value or when a condition of the powder in the vessel changes.

The present summary is provided only by way of example, and not limitation. Other aspects of the present disclosure will be appreciated in view of the entirety of the present disclosure, including the entire text, claims and accompanying figures.

### BRIEF DESCRIPTION OF THE DRAWINGS

FIG. 1 is schematic cross-sectional view of a dry powder fire suppressant monitoring system.
FIG. 2 is an isometric view of one embodiment of a sensor assembly of the dry powder fire suppressant monitoring system of FIG. 1.
FIG. 3 is a perspective top view of the sensor assembly of FIG. 2.
FIG. 4 is a perspective top view of an alternative embodiment of a sensor assembly for the dry powder fire suppressant monitoring system of FIG. 1.

While the above-identified figures set forth embodiments of the present invention, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of the principles of the invention. The figures may not be drawn to scale, and applications and embodiments of the present invention may include features, steps and/or components not specifically shown in the drawings.

### DETAILED DESCRIPTION

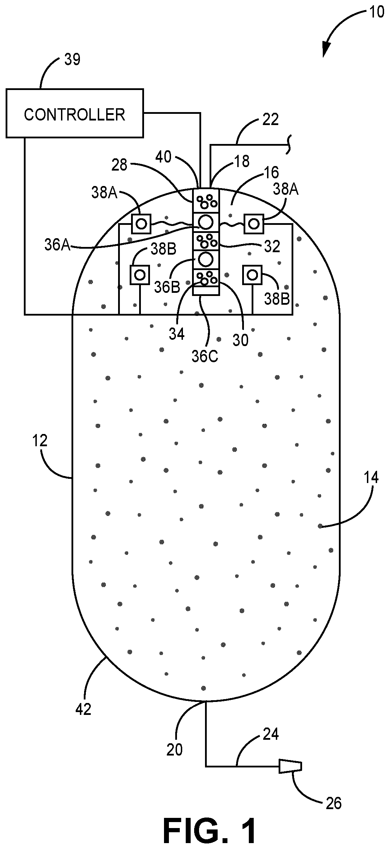

FIG. 1 is simplified schematic cross-sectional view of a dry powder fire suppressant monitoring system combining multiple means for monitoring movement, agglomeration, and/or a volume level of a dry powder in a storage vessel. FIG. 1 shows dry powder fire suppressant monitoring system 10, vessel 12, powder 14, gas 16, inlet 18, outlet 20, conduits 22 and 24, nozzle 26, sensor assembly 28, hollow body 30, wall 32, apertures 34, sensors 36A-36C, 38A, and 38B, and controller 39. Sensor assembly 28 includes hollow body 30 with wall 32 and apertures 34, and sensors 36A-36C.

Dry powder 14, gas 16, sensor assembly 28, and sensors 38A, 38B are contained within an interior volume of vessel 12.

Sensor assembly 28 is disposed in a central region of vessel 12. Sensor assembly 28 is configured to monitor movement, agglomeration, and/or a volume level of powder 14 in the central region of vessel 12.

Sensors 38A and 38B are disposed on an internal wall of vessel 12. Sensors 38A and 38B are configured to monitor movement, agglomeration, and/or a volume level of powder 14 in vessel 12 adjacent to the interior wall. As discussed further herein, fire suppressant monitoring system 10 can include one or both sensor assembly 28 and sensors 38A, 38B.

Controller 39 can be communicatively coupled to sensors 36A-36C and sensors 38A, 38B.

Vessel 12 can include inlet 18 and outlet 20 configured to receive and deliver dry powder 14 and gas 16, respectively. Nozzle 26 can be configured to deliver aerosolized dry powder 14 to an area to be protected from fire.

Dry powder fire suppressant monitoring system 10 (referred to hereinafter as "system 10"), including one or both of sensor assembly 28 and sensors 38A, 38B, is configured for detecting agglomeration of powder 14 stored in fire suppression system vessel 12. System 10 is designed for use on board a vehicle, such as an aircraft, however, is not limited to a particular application. It will be understood by one of ordinary skill in the art that system 10 and the various sensor assemblies and sensor arrangements disclosed herein can be adapted for use with a variety of dry powder fire suppression systems and pressurized dry powder storage vessels used, for example, in industrial or aerospace applications where water is inaccessible or where fire sprinkler systems are not preferred.

System 10 is configured to detect agglomeration of a dry powder fire suppressant in a pressurized storage vessel. Agglomerated dry powder fire suppressant can clog discharge nozzles and conduits thereby rendering a fire suppression system ineffective. Agglomeration of a dry powder fire suppressant can occur during pressurization of a vessel containing the dry powder fire suppressant. For example, if the vessel containing the dry powder is pressurized too rapidly, the powder can be compacted and form an agglomerated mass or smaller agglomerates or clumps of compacted powder particles. Agglomeration of the dry powder can also occur over time as the dry powder settles in the vessel or with changes in ambient temperature and/or pressure.

Vessel 12 is a hollow body configured to contain powder 14 and pressurized gas 16. Vessel 12 can be configured, for example, to contain contents at a pressure equal to or greater than 1200 psi. Vessel 12 can have a spherical shape or generally cylindrical shape with spherical ends as known in the art. The shape of vessel 12 is not limited to the shape illustrated in FIG. 1. Vessel 12 can be formed of any material capable of containing powder 14 and gas 16 at a desired pressure and without reacting with powder 14 and/or gas 16. Vessel 12 can be formed, for example, of stainless steel. Vessel 12 can have a wall thickness rated for a desired pressure.

Vessel 12 can have an inlet 18 and outlet 20. Inlet 18 and outlet 20 can be disposed at opposite ends of vessel 12 as illustrated in FIG. 1. In alternative embodiments, vessel 12 can have a single sealable opening that can serve as both inlet and outlet. Inlet 18 can be configured to receive dry powder 14 and gas 16 through, for example, one or more conduits 22. Outlet 20 can be configured to release dry powder 14 and gas 16 in the event that fire suppression is needed. Outlet 20 can include, for example, a rupture disk configured to release powder 14 and gas 16 upon breaking. Dry powder 14 and gas 16 can be released, for example, through conduit 24. Conduit 24 can be coupled to one or more nozzles 26 disposed in an area to be protected from fire.

Nozzle 26 is configured to deliver dry powder 14 as an aerosol cloud comprising a mixture of fine particles of powder 14 and gas 16. Nozzle 26 can have a small outlet orifice which can be clogged if agglomerates of powder 14 larger than the nozzle outlet orifice are released from vessel 12.

Powder 14 is a dry chemical agent capable of suppressing or extinguishing a fire when delivered in an aerosolized form. Powder 14 can include, for example, sodium bicarbonate, potassium bicarbonate, or monoammonium phosphate. Powder 14 can include coated particles. In some embodiments, powder 14 can be KSA, which is a proprietary finely ground sodium bicarbonate chemical agent developed for aerospace applications. Powder 14 can have a particle size selected for optimal aerosolization and flame extinction. Generally, powder 14 is comprised of micron-sized particles. For example, in some embodiments powder 14 can have an average particle size of equal to or less than 20 microns.

Gas 16 can be nitrogen or another inert gas suitable for fire suppression. Gas 16 fills interstitial spaces between particles of powder 14 and can fill a headspace above powder 14 in vessel 12. Gas 16 can provide the motive force to discharge powder 14 from vessel 12 and nozzle 26 upon opening outlet 20 in the event of a fire.

Powder 14 and gas 16 can be provided to vessel 12 through inlet 18. Vessel 12 can have a vertical orientation extending from top 40 to bottom 42. Inlet 18 can be disposed at top 40 and outlet 20 can be disposed at bottom 42. Powder 14 can settle to bottom 42 in vessel 12. Gas 16 can be supplied to vessel 12 following the addition of powder 14. Gas 16 can be supplied to reach a predefined pressure suitable for discharging powder 14 from vessel 12 and nozzle 26. For example, vessel 12 can be pressurized to approximately 1200 psi with the addition of gas 16. The rate of pressurization or delivery of gas 16 can be selected to minimize the risk of powder compaction and agglomeration. When initially filled for storage and use, powder 14 can fill, for example, approximately 80 to 99 percent of vessel 12. The top approximately 1-20 percent of vessel 12 can be headspace filled with compressed gas 16. The powder volume level (i.e., height of powder 14 in vessel 12 from bottom 42) and headspace volume in the initial fill can be selected to meet a required or suitable volumes of powder 14 and gas 16 for a particular fire suppression application. The powder volume level and headspace volume can also vary over time, with movement of vessel 12 and resulting fluctuations in the powder packing density.

Provided significant compaction and agglomeration does not occur with pressurization of vessel 12, powder 14 remains fluid or able to move or flow in vessel 12 with movement of vessel 12 immediately following pressurization. Over time, powder 14 can settle and compaction and agglomeration can occur.

Sensors 36A-36C, 38A, and 38B are ceramic-based piezoelectric powder level sensors configured to detect the presence and volume level of powder 14 in vessel 12 with high precision. Sensors 36A-36C, 38A, and 38B may also be ablet discern agglomerated powder particles from fine powder particles. Sensors 36A-36C, 38A, and 38B can be, for example, a TSP Series sensor produced by TDK capable of detecting a powder load applied to a sensor face and variation in powder load on the sensor face. TSP Series sensors are oscillation type sensors. An oscillator having a unimorph structure in which a disk-shaped piezoelectric ceramic is adhered to a thin metal plate has full-surface electrodes provided on both sides of the piezoelectric ceramic. An AC voltage applied to the electrodes causes the unimorph to vibrate. When powder 14 contacts the vibrating sensor face, an output signal (e.g., electrical impedance and phase property characteristic) changes according to the powder load, indicating the presence, volume level, and condition (i.e., free flowing or agglomerated) of powder 14 in vessel 12.

Sensors 36A-36C, 38A, and 38B can be communicatively coupled to controller 39. Controller 39 can, for example, convert sensor signals to sensor data, transmit, display, and/or store sensor data, and alert a user when sensors 36A-36C, 38A, and/or 38B indicate a need for maintenance and/or replacement of vessel 12 due to detection of powder agglomeration or conditions favorable for powder agglomeration formation. Controller 39 can be configured, for example, to indicate when a powder volume level falls below a minimum threshold value, the presence of powder is not detected, or when the condition of the powder in the vessel changes. Sensors 36A-36C, 38A, and 38B may use wired or wireless technology for communicating with controller 39.

Sensors 38A and 38B can be disposed on an interior wall of vessel 12. One or more sensors 38A can be disposed in a location of vessel 12 such that the sensor face is positioned at an interface of powder 14 and gas 16 in the headspace. The sensor face of sensors 38A can be partially covered by powder 14. One or more sensors 38B can be disposed in a location of vessel 12 below the powder volume level such that the sensor face is fully disposed in powder 14. In some embodiments, a plurality of sensors 38A can be arranged circumferentially around the interior wall of vessel 12. Each sensor 38A can be disposed an equal distance from top 40 (or bottom 42). In some embodiments, a plurality of sensors 38B can be arranged circumferentially around the interior wall of vessel 12. Each sensor 38B can be disposed an equal distance from top 40 (or bottom 42).

Sensors 38A can be used to monitor changes in powder volume level over time, agglomeration of powder 14, and/or the absence of movement of powder 14 in vessel 12. For example, sensor signals showing steady state powder level or no transient changes in powder load with movement of vessel 12 can indicate agglomeration of powder 14 in vessel 12. Sensor signals indicating a lowered powder level over time even with transient changes in powder load with vessel movement can indicate compaction of power 14, which can increase the risk of agglomeration. In some embodiments, sensors 38A can be calibrated to be able to distinguish agglomerates of powder 14 from fine powder 14. To a lesser extent, compaction of power 14 in vessel 12 may also be detected by sensors 38B as powder load on the sensor face increases with decreasing pore volume.

In some embodiments, controller 39 can be configured to alert a user when sensors 38A (and/or 38B) no longer detect the presence of powder 14 or detect a lowered volume level of powder 14, indicating powder compaction and agglomeration. In some embodiments, controller 39 can be configured to alert a user when sensors 38A detect a reduction in powder load or a powder volume level below a predetermined powder volume level threshold.

In some embodiments, additional sensors (not shown) can be disposed in the headspace above powder 14 to detect movement of powder 14. Movement of powder 14 generally may occur with movement of vessel 12 (e.g., in aircraft maneuvers that involve tilting vessel 12). Sensors disposed in the headspace can be the same as sensors 38A and 38B. Powder load applied to the sensors above the initial powder level may indicate a lower likelihood of powder compaction and agglomeration. As such, sensors positioned in the headspace above powder 14 may be used to confirm that powder 14 remains fluid in vessel 12 and of suitable particle size for use in fire suppression applications.

The number and arrangement of sensors 38A and 38B can be selected to optimize detection of agglomeration of powder 14. As illustrated in FIG. 1, two sensors 38A can be disposed at the same depth from top 40 and can be spaced circumferentially. Two sensors 38B can be disposed in line with and below sensors 38A. In alternative embodiments, sensors 38B can be circumferentially offset from sensors 38A. The two sensors 38B can be disposed at the same depth from top 40 and can be spaced circumferentially. The second sensor 38A and second sensor 38B can be provided for redundancy. Some embodiments can include a single sensor 38A or a single pair of sensors 38A, 38B. Other arrangements are contemplated.

Sensor assembly 28 includes hollow body 30 having wall 32, apertures 34, and sensors 36A-36C. Sensor assembly 28 is configured to monitor movement, agglomeration, and/or the volume level of powder 14 in vessel 12. Sensor assembly 28 positions sensors 36A-36C in the internal void of vessel 12 and spaced apart from the interior wall of vessel 12. Sensors 36A-36C can be disposed away from the interior wall to probe the bulk of powder 14 in the center of vessel 12. Sensor assembly 28 can be attached to top 40 of vessel 12 and extend into vessel 12 in a vertical orientation. Sensor assembly 28 can be centrally located or disposed on an axis of vessel 12.

Sensor assembly 28 can extend a partial depth of vessel 12 between top 40 and bottom 42. For example, sensor assembly 28 can have a length sufficient to position at least one sensor 26A at the interface of powder 14 and gas 16. Sensor assembly 28 can have a length sufficient to position at least one sensor 26B in powder 14 such that a sensor face is fully covered by powder 14.

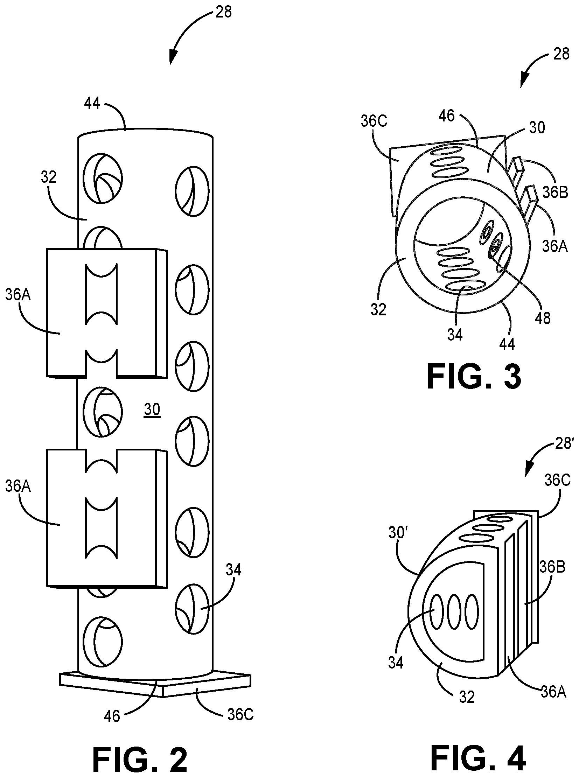

FIG. 2 is an isometric view of sensor assembly 28. FIG. 3 is a perspective top view sensor assembly 28. FIGS. 2 and 3 are discussed together herein. FIG. 2 shows sensor assembly 28 with hollow body 30, wall 32, apertures 34, sensors 36A-36C, top 44, and bottom 46. FIG. 3 shows sensor assembly 28 with hollow body 30, wall 32, apertures 34, sensors 36A-36C, top 44, bottom 46, and sensor face 48.

Hollow body 30 extends longitudinally from top 44 to bottom 46 and is defined by wall 32. Hollow body 30 can be cylindrical. Hollow body 30 can be open at each of top 44 and bottom 46. Hollow body includes a plurality of apertures 34 extending through wall 32. Hollow body 30 can be formed of any material suitable for use in a pressurized environment and that is non-reactive with powder 14.

Hollow body 30 is open to powder 14 in vessel 12 via apertures 34. Hollow body 30 can be partially filled with powder 14. A powder volume level in hollow body 30 can substantially match the powder volume level in vessel 12. A portion of hollow body 30 is disposed in the headspace and open to gas 16.

Sensors 36A-36C can be attached to hollow body 30 such that sensor faces 48 can contact powder 14 in hollow body 30. For example, sensors 36A-36C can be disposed through or in an opening of wall 32. Sensors 36A-36C can be longitudinally spaced. Sensor 36A can be disposed at the interface of powder 14 and gas 16. Sensors 36B and 36C can be disposed below the powder level such that sensor faces 48 are fully covered by powder 14. Sensor 36C can be disposed on bottom 46 of hollow body and perpendicular to sensors 36A and 36B such that a full weight of powder 14 is provided on a sensor face of sensor 36C.

Sensors 36A-36C can detect the presence of powder 14 and changes in powder load or powder volume level as described with respect to sensors 38A and 38B. As previously discussed, sensor 36A can be used to monitor a powder volume level or load and changes in powder volume level or load over time. To a lesser extent, sensors 36B and 36C may be used to monitor changes in powder load or powder compaction over time. As previously discussed, reduction in the powder volume level or an unchanging powder volume level with movement of vessel 12 can indicate compaction of powder 14 and formation of agglomerates.

In alternative embodiments, sensor 36A can be disposed in the headspace above powder 14 and sensor 36B can be disposed at the interface of powder 14 and gas 16. As previously described, a sensor positioned in the headspace above the powder level can be used to monitor movement of powder 14 in vessel 12 and hollow body 30.

Apertures 34 can allow powder 14 to move between vessel 12 and the interior of hollow body 30. In some embodiments, apertures 34 can be sized to prevent migration of agglomerates of powder 14 having a size greater than the discharge orifice of nozzle 26 (shown in FIG. 1). Agglomerated powder 14 in vessel 12 can thereby be prevented from moving into hollow body 30 and across sensor 36A. As such, sensor 36A can be used to distinguish fine powder particles and agglomerates of powder 14 that are still flowable in vessel 12 but smaller than the outlet orifice of nozzle 26 due to agglomerates of powder 14 that are too large for discharge from vessel 12. In one non-limiting embodiment, apertures can have a diameter equal to or less than 1/16 inch (approximately 1.6 mm) for systems 10 having a nozzle outlet orifice of 1/16 inch (approximately 1.6 mm).

Apertures 34 can be arranged around hollow body 30 from top 44 to bottom 46. Apertures 34 can be arranged in any manner suitable for promoting flow through hollow body 30.

FIG. 4 is a perspective top view of an alternative embodiment of a sensor assembly 28. FIG. 4 shows sensor assembly 28', hollow body 30', wall 32', apertures 34, and sensors 36A-36C. Sensor assembly 28' is similar to sensor assembly 28 with the exception that hollow body 30' is semicylindrical in shape defined by wall 32'. Sensor assembly 28' includes sensors 36A-36C, which can be disposed on a flat surface of hollow body 30'. Sensors 36A-36C can be arranged as described with respect to sensor assembly 28.

Sensor assemblies 28 and 28' are two examples of sensor assemblies that can be used to detect powder 14 and monitor powder movement, agglomeration, and/or powder volume level within vessel 12. Other hollow body shapes, sensor arrangements, and aperture configurations are also contemplated in view of this disclosure. It will be understood by one of ordinary skill in the art that elements of sensor assemblies 28 and 28' are not drawn to scale and that the embodiments disclosed herein are intended to provide an explanation of the present invention and not a limitation of the invention. The present invention is not limited to the embodiments disclosed. It will be understood by one skilled in the art that various modifications and variations can be made to the invention without departing from the scope and spirit of the invention.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

### Discussion of Possible Embodiments

The following are non-exclusive descriptions of possible embodiments of the present invention.

An assembly for detecting agglomeration of a powder for a fire suppression system includes a vessel configured to contain a powder and a compressed gas and a plurality of piezoelectric sensors disposed in the vessel. At least one sensor of the plurality of sensors is disposed at an interface of the powder and the compressed gas when the vessel is in a filled state.

The assembly of the preceding paragraph can optionally include, additionally and/or alternatively, any one or more of the following features, configurations and/or additional components:

In an embodiment of the foregoing assembly, the at least one sensor can be disposed on an interior wall of the vessel.

In an embodiment of any of the foregoing assemblies, the at least one sensor can be spaced apart from the interior wall.

An embodiment of any of the foregoing assemblies can further include a hollow body disposed in the vessel. The hollow body can have a plurality of apertures configured to allow a flow between the vessel and an interior of the hollow body. At least one sensor can be disposed in the interior of the hollow body.

In an embodiment of any of the foregoing assemblies, the hollow body can be positioned near a top of the vessel.

In an embodiment of any of the foregoing assemblies, the hollow body can extend longitudinally from a top to a bottom and the plurality of sensors can be longitudinally spaced.

In an embodiment of any of the foregoing assemblies, the hollow body can be disposed in a center of the vessel.

In an embodiment of any of the foregoing assemblies, the hollow body can be cylindrical or semicylindrical.

In an embodiment of any of the foregoing assemblies, the hollow body can extend longitudinally from a top to a bottom. The at least one sensor can be a first sensor. A second sensor of the plurality of sensors can be disposed at the bottom of the hollow body and oriented perpendicular to the first sensor.

In an embodiment of any of the foregoing assemblies, the apertures can have a diameter equal to or less than a diameter of a nozzle outlet through which the powder is delivered upon being discharged from the vessel.

A dry powder fire suppressant monitoring system includes a vessel configured to contain a powder and a compressed gas, a plurality of piezoelectric sensors disposed in the vessel, and a controller communicatively coupled to the plurality of sensors. At least one sensor of the plurality of sensors is disposed at an interface of the powder and the compressed gas when the vessel is in a filled state. The controller is configured to receive an output from the plurality of sensors that corresponds to powder load, indicating at least one of a presence, a volume level, and a condition of the powder in the vessel, and the controller configured to indicate when a powder volume level falls below a minimum threshold value (or the presence of powder is not detected) or when a condition of the powder in the vessel changes.

The system of the preceding paragraph can optionally include any one or more of the following features, configurations and/or additional components:

The at least one sensor can be disposed on the interior wall.

The at least one sensor can be spaced apart from the interior wall.

The system can further include a hollow body disposed in the vessel. The hollow body can have a plurality of apertures configured to allow a flow of powder between the vessel and an interior of the hollow body. The at least one sensor can be disposed in the interior of the hollow body.

The hollow body can be positioned near a top of the vessel.

The hollow body can extend longitudinally from a top to a bottom and the plurality of sensors can be longitudinally spaced.

The hollow body can be disposed in a center of the vessel.

The hollow body can be cylindrical or semicylindrical.

The hollow body can extend longitudinally from a top to a bottom. The at least one sensor can be a first sensor and a second sensor of the plurality of sensors can be disposed at the bottom of the hollow body and oriented perpendicular to the first sensor.

The apertures can have a diameter equal to or less than a diameter of a nozzle through which the powder is delivered upon being discharged from the vessel.

## Claims

1. An assembly for detecting agglomeration of a powder for a fire suppression system, the assembly comprising:
a vessel configured to contain a powder and a compressed gas, the vessel having an interior wall; and
a plurality of sensors disposed in the vessel, wherein at least one sensor of the plurality of sensors is disposed at an interface of the powder and the compressed gas when the vessel is in a filled state, and wherein the sensors of the plurality of sensors are piezoelectric sensors.

2. The assembly of claim 1, wherein the at least one sensor is disposed on the interior wall.

3. The assembly of claim 1 or 2, wherein the at least one sensor is spaced apart from the interior wall.

4. The apparatus of claim 3 and further comprising a hollow body disposed in the vessel, the hollow body having a plurality of apertures configured to allow a flow between the vessel and an interior of the hollow body, wherein the at least one sensor is disposed in the interior of the hollow body.

5. The assembly of claim 4, wherein the hollow body is positioned near a top of the vessel.

6. The assembly of claim 4 or 5, wherein the hollow body extends longitudinally from a top to a bottom and wherein the plurality of sensors are longitudinally spaced.

7. The assembly of claim 4, 5 or 6, wherein the hollow body is disposed in a center of the vessel.

8. The assembly of any one of claims 4-, wherein the hollow body is cylindrical or semicylindrical.

9. The assembly of any one of claims 4-8, wherein the hollow body extends longitudinally from a top to a bottom and wherein the at least one sensor is a first sensor and wherein a second sensor of the plurality of sensors is disposed at the bottom of the hollow body and oriented perpendicular to the first sensor.

10. The assembly of any one of claims 4-9, wherein the apertures have a diameter equal to or less than a diameter of a nozzle outlet through which the powder is delivered upon being discharged from the vessel.

11. The assembly of any preceding claim, wherein the at least one sensor is a first sensor and wherein a second sensor of the plurality of sensors is disposed in a location of the vessel below a powder volume level such that a sensor face of the second sensor is fully disposed in powder when the vessel is in a filled state.

12. The assembly of any preceding claim, wherein the at least one sensor comprises a plurality of sensors disposed at an interface of the powder and the compressed gas, the plurality of sensors arranged circumferentially around the interior wall and disposed an equal distance from a top of the vessel.

13. A dry powder fire suppressant monitoring system comprising:
an assembly as claimed in any preceding claim; and
a controller communicatively coupled to the plurality of sensors, the controller configured to receive an output from the plurality of sensors, the output corresponding to a powder load, indicating at least one of a presence, a volume level, and a condition of the powder in the vessel, and the controller configured to indicate when a powder volume level falls below a minimum threshold value, the presence of powder is not detected, or when a condition of the powder in the vessel changes.
